# EUROPEAN PATENT APPLICATION

(11) **EP 1 669 748 A1**
(43) Date of publication of application: **14.06.2006**
(21) Application number: 04772319.2
(22) Date of filing: 27.08.2004
(51) Int. Cl.: G01N 27/414, G01N 27/00

(54) **BIOMOLECULE DETECTING ELEMENT AND METHOD FOR ANALYZING NUCLEIC ACID USING THE SAME**

(30) Priority: 29.08.2003 JP 2003306906
(71) Applicant: National Institute for Materials Science, Tsukuba-shi, Ibaraki 305-0047 (JP)
(72) Inventor: Miyahara, Yuji, Nat. Inst. for Materials Science, Tsukuba-shi, Ibaraki 305-0047 (JP); Sakata, Toshiya, Nat. Inst. for Materials Science, Tsukuba-shi, Ibaraki 305-0047 (JP); Kamahori, Masao, c/o Central Research Laboratory, Kokubunji-shi, Tokyo 185-8601 (JP); Yazawa, Yoshiaki, c/o Central Research Laboratory, Kokubunji-shi, Tokyo 185-8601 (JP)
(74) Representative: Schrell, Andreas
(86) International application number: PCT/JP2004/012363
(87) International publication number: WO 2005/022142

(57) **Abstract**

An inexpensive DNA chip/DNA microarray system capable of highly accurate measurement with low running cost. A DNA probe 8 is immobilized on a floating electrode 7 connected to a gate electrode 5 of a field effect transistor. Hybridization with a target gene is carried out on the surface of the floating electrode, when a change in surface charge density is detected using a field effect.

## Description

### TECHNICAL FIELD

The present invention relates to biotechnology, particularly technologies in the field of genetic examination, such as genetic diagnosis, DNA sequencing analysis, and gene polymorphism analysis. In particular, the invention relates to a biomolecule detecting element suitable for accurately analyzing a plurality of different nucleic acids in parallel, and to a method for analyzing nucleic acids employing the element.

### BACKGROUND ART

With the rapid increase in the rate at which the genome sequences of various living organisms, such as the human genome, are read, vast amounts of base sequence data have been accumulated. It is expected that from now on, gene functions in living organisms will be clarified and a significant progress will be made in gene-related technologies in a wide array of fields, such as in the diagnosis of a variety of diseases, development of drugs, and improvements in agricultural product varieties. These developments in new fields will be based on gene expression and function data, as well as base sequence data. As a technology for carrying out a gene function and expression analysis on a large scale so as to enable gene examinations, DNA chips or DNA microarrays have been developed by Affymetrix Inc. and Nanogen, Inc., for example. However, many of the existing DNA chips or DNA microarrays are based on the detection of fluorescence as a basic principle, so that they require lasers or complex optical systems, resulting in an increase in the size and cost of the system.

In order to solve these problems, several DNA chips of a current detection type have been reported, in which a redox marker is used in combination. For example, Clinical Micro Sensors Inc. has developed a system in which one end of a molecule called a molecular wire is immobilized on a metal electrode, and a DNA probe is bound to the other end. Transfer of electrons between the redox marker and the metal electrode based on the hybridization with a target gene is detected in the form of a current change, thereby detecting the target gene (Nature Biotechnology, vol. 16, (1998), p.27 and p.40).

### Non-patent Document 1: Nature Biotechnology, vol. 16, (1998), p.27 and p.40

### DISCLOSURE OF THE INVENTION

### Problems to be Solved by the Invention

Because many of the current DNA chips/DNA microarrays are based on the detection of fluorescence as a basic principle, they require lasers or complex optical systems, resulting in an increase in the size and cost of the system. Further, all of the currently developed DNA chips/DNA microarrays are discarded after just one use as a rule. If they are washable and reusable, they could be used up to twice or three times at most. As a result, in the field of analysis involving many samples or gene diagnosis involving many specimens, the problem of high running cost cannot be disregarded. Particularly in the medial field, widespread use of expensive examinations would be unlikely from the viewpoint of cost reduction. On the other hand, in the field of medical diagnosis, namely in gene diagnosis, high levels of accuracy and quantitativity are required. Therefore, there is a need for a technology that can satisfy the need for both cost reduction and high accuracy.

Because the aforementioned electrochemical detection method does not require expensive lasers or complex optical systems, systems can be reduced in size and cost as compared with systems based on the fluorescent detection method. However, this method is based on a redox reaction on a metal electrode as a basic detection principle, if an oxidizing substance or a reducing substance (such as ascorbic acid) exists in the sample, a current based on oxidation or reduction flows, which would hinder the gene detection and the detection accuracy would deteriorate. Furthermore, the measurement of current is associated with the progress of electrode reaction on the metal electrode. Because such an electrode reaction is irreversible and constitutes a nonequilibrium reaction, corrosion of the electrode develops and gases are generated, for example. As a result, the stability of current measurement is adversely affected and the detection accuracy deteriorates particularly when measurement is repeated.

It is therefore an object of the invention to provide an inexpensive DNA chip/DNA microarray system capable of high accuracy measurement at low running cost.

### Means for Solving the Problem

In accordance with the invention, a probe comprising a living body-related substance, such as nucleic acid, is immobilized on the surface of a metal that is electrically connected to the gate of a field effect transistor, and then a complex with a target substance is formed on the metal surface when a change in surface charge density is detected using a field effect. In addition to the charge possessed by the living body-related substance, an intercalator is introduced or the complex is combined with a charged particle, such as an ion, so that the amount of change in surface charge density can be increased and the change in surface charge potential can be detected with a large S/N ratio.

Meanwhile, wireless communications technologies have made a significant progress in recent years, and a wireless communications chip has been developed in which a tag that is equivalent to a barcode is integrated. In this technology, individual wireless communications chips can be identified, and their use as a distribution management chip is being considered. When such a tagged wireless communications chip is used as a DNA chip, it must be of low-power consumption because electric power is fed wirelessly. The voltage measurement system, which consumes less power than the current measurement system, is therefore suitable for the wireless communications-based DNA chip.

The invention provides a biomolecule detecting element comprising:
an insulated gate field effect transistor having a gate electrode embedded in an insulating film;
a probe-immobilized electrode formed on the surface of the insulating film and having a biomolecular probe immobilized thereon; and
a connection wire for electrically connecting the gate electrode and the probe-immobilized electrode,
wherein the region of the probe-immobilized electrode where the biomolecular probe is immobilized is located away from immediately above the gate electrode.

In a preferred embodiment, the probe-immobilized electrode extends from immediately above the gate electrode along the film surface of the insulating film to the region where the biomolecular probe is immobilized,
wherein the connection wire is connected to the probe-immobilized electrode immediately above the gate electrode. In another embodiment, the probe-immobilized electrode is disposed away from immediately above the gate electrode, and the connection wire is disposed within the insulating film along the film surface.

The biomolecular probe may comprise a nucleic acid, polynucleotide, or synthetic oligonucleotide. The biomolecular probe has one end thereof immobilized on the surface of the probe-immobilized electrode and specifically binds to and/or reacts with a living body-related substance in a sample. The biomolecular probe may be comprised of a single-strand probe, and detection sensitivity with respect to a specific binding between the probe and a complementary strand can be enhanced by placing an intercalator at the double-stranded portion formed by the specific binding. The probe-immobilized electrode may be comprised of one or a combination of materials including gold, platinum, palladium, titanium, chromium, aluminum, polysilicon, tantalum, or molybdenum. A transmission/reception antenna may be formed in the insulating film.

The invention also provides a biomolecule detecting element comprising:
a plurality of insulated gate field effect transistors each having a gate electrode embedded in a common insulating film;
a plurality of probe-immobilized electrodes formed on the surface of the insulating film and having biomolecular probes immobilized thereon; and a plurality of connection wires for electrically connecting the gate electrode of each of the plurality of insulated gate field effect transistors and the plurality of probe-immobilized electrodes,
wherein the region of the probe-immobilized electrodes where the biomolecular probes are immobilized is located away from immediately above the gate electrode. A transmission/reception antenna may be formed in the common insulating film. Preferably, a computation circuit, a memory circuit, a reception circuit, a transmission circuit, and a power supply circuit are also provided. In this case, the power supply circuit preferably converts the electromagnetic wave received by the antenna into electric power and feeds it to individual portions. These circuits can be produced by the existing technologies.

The invention also provides a method for analyzing nucleic acids using the biomolecule detecting element of the invention, comprising the steps of:
immobilizing a single-stranded nucleic acid probe on the probe-immobilized electrode as a biomolecular probe;
introducing a sample solution containing at least one kind of nucleic acid onto the biomolecule detecting element and carrying out hybridization with the single-stranded nucleic acid probe;
introducing a washing solution onto the biomolecule detecting element and removing unreacted nucleic acid on the biomolecule detecting element;
introducing an intercalator solution onto the biomolecule detecting element and reacting it with the nucleic acid that has become double-stranded;
introducing a washing solution onto the biomolecule detecting element and removing unreacted intercalator on the biomolecule detecting element; and
introducing a buffer solution onto the biomolecule detecting element and measuring output values of the insulated gate field effect transistor.

The invention further provides a method for analyzing nucleic acids using the biomolecule detecting element of the invention, comprising the steps of:
putting a plurality of biomolecule detecting elements comprising the probe-immobilized electrodes having different kinds of single-stranded nucleic acid probes immobilized thereon as biomolecular probes, and a buffer solution in a reaction vessel, and receiving a signal from each of the biomolecule detecting elements using an external receiver;
introducing a sample solution containing at least one kind of nucleic acid into the reaction vessel and carrying out hybridization with the single-stranded nucleic acid probe;
introducing an intercalator solution into the reaction vessel and causing it to react with the nucleic acid that has become double-stranded; and
receiving a signal from each of the biomolecule detecting elements using an external receiver.

### Effect of the Invention

The biomolecule detecting element of the invention does not require expensive lasers or complex optical detection systems. Because the biomolecule detecting element of the invention is based on the detection of surface potentials in a steady state, as opposed to the current detection-based (amperometric) system, there is no problem of instability in signal values due to the corrosion of substrates or the development of gas, or the interference by oxidizing and/or reducing substances. Thus, detection of biological material can be made highly accurately and stably.

### BRIEF DESCRIPTION OF THE DRAWINGS

Fig. 1 shows a biomolecule detecting transistor employing a field effect transistor and a floating-gate electrode according to the invention.
Fig. 2 shows a gene detection circuit employing the biomolecule detecting transistor of the invention.
Fig. 3 shows a system combining the biomolecule detecting transistor of the invention and an intercalator.
Fig. 4 shows how the biomolecule detecting transistor of the invention and an antenna are integrated.
Fig. 5 shows a planar arrangement of the biomolecule detecting transistor of the invention and an antenna.
Fig. 6 shows a system in which the biomolecule detecting transistor of the invention and a wireless communications chip are integrated.
Fig. 7 shows a gene analysis system employing the biomolecule detecting transistor of the invention.

### BEST MODE FOR CARRYING OUT THE INVENTION

Embodiments of the invention will be described with reference to the drawings. In the following, DNA is used as an example of a biomolecule.

### Embodiment 1

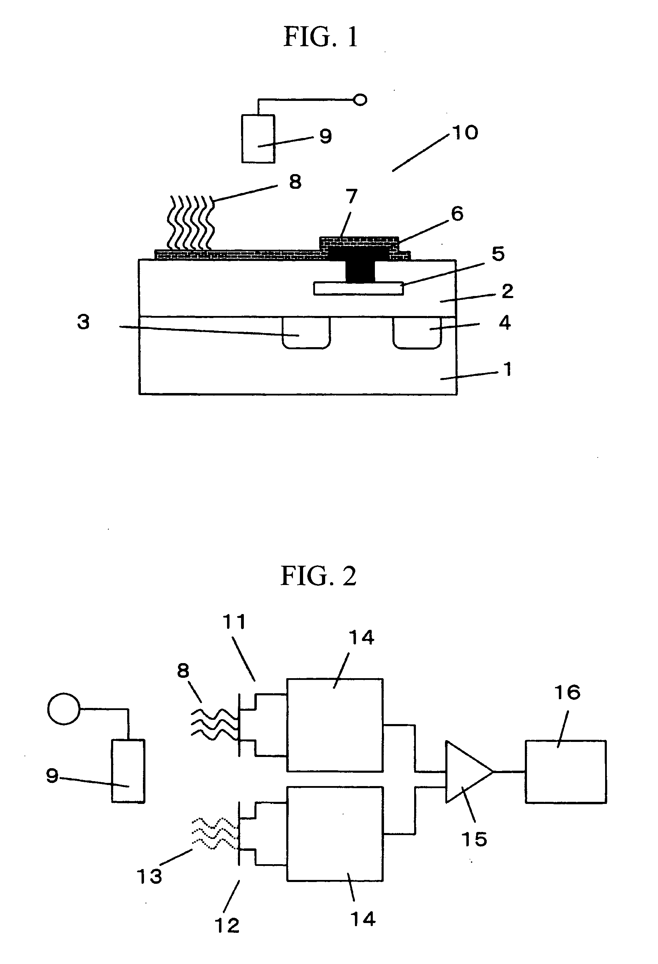

Fig. 1 schematically shows a cross section of a biomolecule detecting element (biomolecule detecting transistor) according to the invention.

An insulated gate field effect transistor is produced by forming a gate insulating film 2, a source 3, and a drain 4 on the surface of a silicon substrate 1, and providing a gate electrode 5 on the gate insulating film between the source and drain. On the surface of the gate electrode 5 is further formed an insulating film, such that the gate electrode 5 is embedded in the insulating film 2. A throughhole is formed in the insulating film 2, and a lead-out electrode 6 is formed using a conductive material and is placed in an electrical contact with the gate electrode 5. A floating electrode 7 is further formed on the surface of the gate insulating film and is placed in electrical contact with the lead-out electrode 6. A DNA probe 8 is immobilized on the surface of the floating electrode 7. The thus produced gene transistor is immersed in a sample solution 10 together with a reference electrode 9 when in use.

The gate insulating film is comprised of silicon oxide (SiO₂), silicon nitride (SiN), aluminum oxide (Al₂O₃), or tantalum oxide (Ta₂O₅), either individually or in combination. Normally, in order to ensure satisfactory transistor operation, silicon nitride (SiN), aluminum oxide (Al₂O₃), or tantalum oxide (Ta₂O₅) is layered on the silicon oxide (SiO₂), thereby producing a double-layered structure. The material of the gate electrode 5 is preferably polysilicon because it provides good compatibility with the so-called self-align process, in which the source and drain are formed through the injection of ions via a polysilicon gate. The lead-out electrode 6 is used as part of wiring and is therefore preferably made of a material with low resistance and good etching processibility, for example. Examples include polysilicon, aluminum, and molybdenum. The floating electrode 7, which comes into direct contact with a sample solution, is preferably made of a material that is highly chemically stable and that exhibits a stable potential, and which has high affinity to biological materials to which the floating electrode is fixed. Examples include noble metals such as gold, platinum, silver, and palladium. The lead-out electrode and the floating electrode can be formed of the same material, such as gold, by using a pattern forming method for the floating electrode, such as the liftoff method.

In accordance with the biomolecule detecting transistor of the present embodiment, the need for fixing the DNA probe 8 on the channel between the source and drain of the transistor is eliminated, so that the DNA probe 8 can be formed on any desired location on the chip by extending the floating electrode 7 or the lead-out electrode 6, as shown in Fig. 1. As a result, the region where the DNA probe 8, which comes into contact with the sample solution, for example, is formed and the transistor region where electronic circuits are formed can be laid out separately on the chip, which enables highly reliable measurement. Normally, in chips for biomolecule detection purposes, it is necessary to immobilize different biomolecules separately. For this reason, such biomolecule detecting chips are larger in size than the conventional semiconductor chips, and biomolecule detecting chips have even been developed that are as large as a slide glass (26 mm x 76 mm). While it is preferable to design the biomolecule detecting transistor of the invention to be of a small size of approximately 5 mm squares for price reduction purposes, the concept of the invention may be applied to chips whose size is about the size of a slide glass.

The DNA probe (biomolecular probe) 8 is normally comprised of not more than 300 bases, using fragments of oligonucleotide or cDNA. When oligonucleotide is used, the nucleic acid fragment is preferably not more than 80 bases long. In order to immobilize the DNA probe 8 on the surface of the floating gate electrode 7, one end of the DNA probe is chemically modified with an amino group (NH₂ group), a thiol group (SH group), or biotin, for example. When a DNA probe modified with an amino group is used, the surface of the gate electrode is chemically modified with aminopropylethoxysilane or polylysine, for example, thereby introducing an amino group on the gate surface. A DNA probe chemically modified with an amino group through a reaction with glutaraldehyde or phenylenediisocyanate (PDC) is then immobilized on the gate surface. When a DNA probe chemically modified with a thiol group is immobilized on the gate electrode surface, gold is used for the gate electrode, and the DNA probe is immobilized using the affinity between the thiol group and gold. Further, when a DNA probe chemically modified with biotin is immobilized, streptavidin is introduced on the gate electrode surface, and the DNA probe is immobilized on the gate surface using the affinity between the biotin and streptavidin. When the DNA probe is immobilized in practice, a solution containing the DNA probe is added dropwise or spotted onto the floating electrode surface alone.

In order to stably measure the potential change caused by the chemical reaction that takes place on the gate electrode surface of the biomolecule detecting transistor, a reference electrode 9 is disposed that provides a reference for potential measurement. The reference electrode is normally prepared by immersing a silver/silver chloride electrode or a photosensitive electrode in an internal solution of a predetermined composition and concentration. Because the operating point is adjusted by changing the electric characteristics of the biomolecule detecting transistor, a predetermined voltage can be applied to the reference electrode 9.

If there are many genes in the sample including the target gene to be measured and if the DNA probe immobilized on the gate of the biomolecule detecting transistor has a base sequence complementary to the target gene, the target gene and the DNA probe hybridize under proper reaction conditions and form a complex. Under proper pH condition of the buffer solution used for the reaction, DNA is charged negatively. Therefore, the formation of a complex by hybridization leads to a change in charge density near the gate of the FET, which causes a change in the gate surface potential. This change produces an action equivalent to a gate voltage change in the FET, whereby the channel conductivity changes. Thus, the formation of the complex, namely the presence of the target gene, can be detected via the change in drain current through the source 3 and the drain 4.

Gene analysis is performed using the biomolecule detecting transistor of the present embodiment in accordance with the following procedure, for example.

Initially, the biomolecule detecting transistor of the invention and 0.5 ml of buffer solution are put in a reaction vessel, and transistor signals are measured. Thereafter, gene analysis is performed in the following steps (a) to (e).
(a) A sample solution containing at least one kind of DNA is introduced into the reaction vessel, and the DNA is hybridized with the single-strand DNA probe on the conductive electrode at a predetermined temperature.
(b) A washing solution is introduced into the reaction vessel, and unreacted DNA on the substrate is removed.
(c) An intercalator solution is introduced into the reaction vessel and caused to react with the DNA that has become double-stranded.
(d) A washing solution is introduced into the reaction vessel and unreacted intercalator on the substrate is removed.
(e) A buffer solution is introduced into the reaction vessel, and output values of the insulated gate field effect transistor are measured.

For simplifying and expediting the measurement, the steps (b) and (d) may be skipped.

### Embodiment 2

Fig. 2 schematically shows a gene examination system in which the biomolecule detecting transistor according to the first embodiment is used. In this system, a reference transistor 12 is used in addition to the biomolecule detecting transistor 11 shown in Fig. 1, and a differential measurement is carried out using the two transistors.

On the surface of the gate of the biomolecule detecting transistor, a DNA probe 8 having a base sequence complementary to that of the target gene in the sample is immobilized. On the other hand, on the surface of the gate of the reference transistor, a DNA probe 13 having a base sequence different from the complementary base sequence of the target gene is immobilized. To stably measure the surface potential of the biomolecule detecting transistor and the reference transistor, a reference electrode 9 as a reference for potential measurement is provided. The surface potential of the biomolecule detecting transistor and that of the reference transistor are measured by a drive circuit 14, and a measurement signal is fed to a signal processing circuit 16 via a differential measuring circuit 15.

By thus carrying out a differential measurement, changes in output values caused by different electric characteristics of transistors in response to changes in ambient temperature or optical conditions can be compensated. Furthermore, the changes in output values due to the nonspecific adsorption of the charged particles in the sample on the gate can also be compensated, thereby allowing the change in output due to hybridization between the target gene and the DNA probe alone to be exclusively detected with high accuracy.

The biomolecule detecting transistor and the reference transistor should desirably have identical electric characteristics. Therefore, a pair of transistors integrated on the same substrate are preferably used. When a plurality of biomolecule detecting transistors are integrated so as to measure a plurality of genes simultaneously, the reference transistor may be commonly used. In this case, the different biomolecule detecting transistors and the common reference transistor are used for a differential measurement.

### Embodiment 3

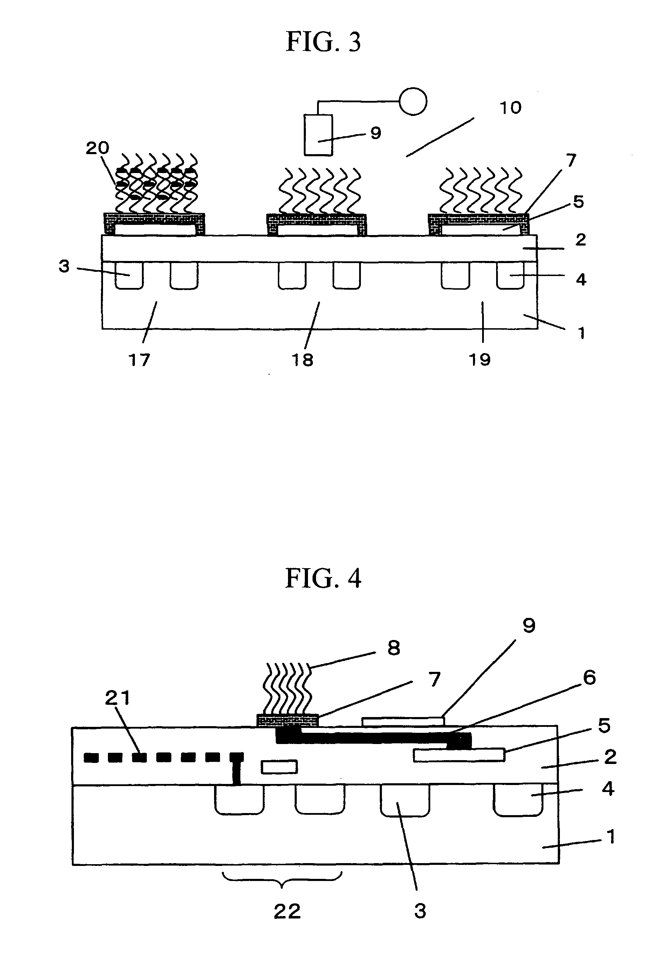

Fig. 3 schematically shows a cross section of another example of a measurement system employing the biomolecule detecting transistor shown in Fig. 1. In this measurement system, three FETs are integrated. A first biomolecule detecting transistor 17 is used for detecting a first target gene. A second transistor 18 is used for detecting a second target gene. A third transistor 19 is used as the reference transistor. On the gate electrode of the first and second biomolecule detecting transistors, a DNA probe having a base sequence complementary to that of the first and the second gene, respectively, is immobilized. On the surface of the gate electrode of the reference FET, a DNA probe having a base sequence different from the complementary base sequence of the first or second gene is immobilized.

The state shown in Fig. 3 is that where a sample solution containing only the first gene has been introduced into the aforementioned integrated transistors and hybridized to the target gene, and then an intercalator has been added. The first gene hybridizes to the DNA probe of the first biomolecule detecting transistor 17 alone, thereby forming a double strand. The intercalator 20 reacts with and binds to the double-stranded DNA alone, without binding to single-strand DNA. Because the intercalator has a charge, the surface charge density of the first biomolecule detecting transistor 17 alone changes, resulting in a change in its output signal. The surface charge density of the second biomolecule detecting transistor 18 and that of the reference transistor 19 do not change, so that there is no change in their output signals. Therefore, by performing a differential measurement between the first biomolecule detecting transistor 17 and the reference transistor 19, and between the second biomolecule detecting transistor 18 and the reference transistor 19, the output signal of the former alone changes, whereby the first target gene can be detected. As the intercalator, ethidium bromide, Hoechst 33258, or PicoGreen, for example, may be used.

An example of measurement will be described in the following. Alcohol dehydrogenase-related genes are known to have single nucleotide polymorphisms (SNPs). A first and a second DNA probe of 17-bases long each consisting of 8 bases in front and 8 bases after an SNP site were synthesized. The base sequences are as follows:
First DNA probe: 5'-CATACACTAAAGTGAAA-3' (SEQ ID NO:1)
Second DNA probe: 5'-CATACACTGAAGTGAAA-3' (SEQ ID NO:2)
The ninth site from the 5' terminal is the SNP site, which is A in the case of the first DNA probe and G in the case of the second probe. The first and second DNA probes were immobilized on a floating electrode connected to the gate of the first and second transistors, respectively. For the immobilization of the DNA probes, the 5'-terminal of the DNA probe was modified with a thiol group. The floating electrode connected to the gate of the FET in the present embodiment is comprised of a gold floating electrode, on the surface of which the aforementioned DNA probes were immobilized. To the floating electrode connected to the gate of the reference transistor, a DNA probe having a sequence different from that of the first or second DNA probe, namely a DNA probe of 17-bases long consisting solely of As, was synthesized and immobilized. Alternatively, no DNA probe may be immobilized on the floating electrode connected to the gate of the reference transistor.

As a sample, human genome was extracted from the white blood cells in blood, and a 100-base length region including the aforementioned SNP site was amplified. Thereafter, the region was introduced into the first and second biomolecule detecting transistors and the reference transistor, and then hybridization was carried out at 45°C for 8 hours. After hybridization, washing was carried out using a buffer solution so as to remove unreacted sample, followed by the introduction of intercalator. For measurement, a buffer solution was introduced into the first and second biomolecule detecting transistors and the reference transistor, and the output voltage of each transistor, a differential output between the first biomolecule detecting transistor and the reference transistor, and a differential output between the second biomolecule detecting transistor and the reference transistor were measured. Thereafter, the sample was introduced, followed by hybridization and washing. Hoechst 33258 was then introduced as the intercalator, and the output voltage of each transistor, a differential output between the first biomolecule detecting transistor and the reference transistor, and a differential output between the second biomolecule detecting transistor and the reference transistor were measured. In this way, changes in output voltage before and after the introduction of the sample and intercalator were measured.

The result of measurement was as follows. In the case of the sample (Normal) having a base sequence corresponding to that of the first DNA probe, the differential output between the first biomolecule detecting transistor and the reference transistor after the introduction of the sample solution and after the introduction of intercalator was 15.0 mV and -12.0 mV, respectively. Because DNA is negatively charged in the solution, the output of an n-channel FET is shifted in the positive direction. On the other hand, because the intercalator is positively charged in the solution, the output of the FET is shifted in the negative direction. The differential output between the second biomolecule detecting transistor and the reference transistor was 1.5 mV and
- 0.5 mV after the introduction of the sample solution and after the introduction of intercalator, respectively, thus indicating a significant difference. The intercalator reacts solely with the double-stranded DNA and has a charge opposite to the DNA, so that the intercalator does not respond to the single-strand DNA nonspecifically adsorbed on the floating electrode. Thus, the signal due to the nonspecific adsorption of the single-stranded DNA and the signal due to hybridization based on the double-stranded DNA can be clearly distinguished.

In the case of a sample (Mutant) having a base sequence corresponding to that of the second DNA probe, the differential output between the first biomolecule detecting transistor and the reference transistor after the introduction of the sample solution and after the introduction of intercalator was 2.3 mV and 0.7 mV, respectively. On the other hand, the differential output between the second biomolecule detecting transistor and the reference transistor was 11.0 mV and -8.0 mV, respectively, thus also indicating a significant difference.

In the case of a sample (hetero) having half of the base sequence of the first DNA probe and half of the base sequence of the second DNA probe, the differential output between the first biomolecule detecting transistor and the reference transistor after the introduction of the sample solution and after the introduction of intercalator was 6.5 mV and -4.8 mV, respectively. On the other hand, the differential output between the second biomolecule detecting transistor and the reference transistor was 5.5 mV and -4.5 mV, respectively, thus indicating an almost one to one ratio.

Thus, by performing an SNP analysis in accordance with the invention, three kinds of samples, namely, a Normal/Normal homo, a Mutant/Mutant homo, and a Normal/Mutant hetero, were successfully identified. When an intercalator is used, there is no need to chemically bind a label compound to the sample DNA for labeling purposes.

### Embodiment 4

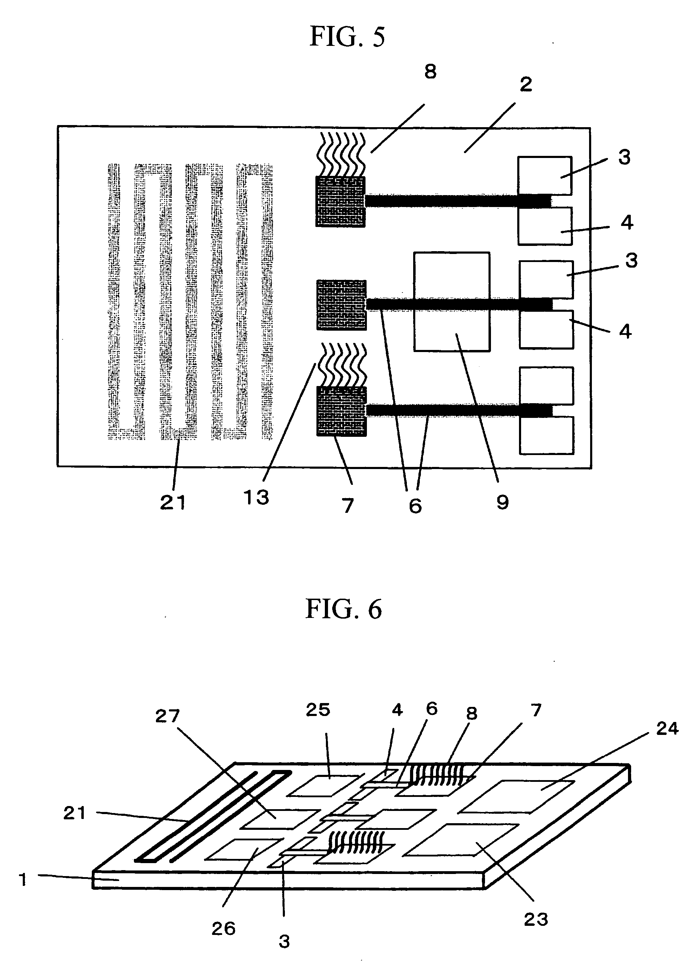

Figs. 4 and 5 schematically show another example of the biomolecule detecting transistor according to the invention. The biomolecule detecting transistor is equivalent to the biomolecule detecting transistor shown in Fig. 3 to which a transmission/reception antenna 21 has been added in the gate insulating film 2. The antenna 21, which may be formed simultaneously with the formation of the gate electrode 5, is connected to an element 22 of a transmission/reception circuit embedded in chip. In the present embodiment, a reference electrode 9 is comprised of Ti on which Pt is stacked and is directly formed on a substrate.

In the present embodiment, a lead-out electrode 6 is embedded in an insulating film 2 and extended therein, and a floating electrode 7 is formed at the end thereof. Fig. 5 shows a plan view of the embodiment. Source 3 and drain 4 of the transistor, DNA probes 8 and 13, and antenna 21 can be separately laid out on the chip. The only portions exposed on the chip surface are the floating electrode 7, DNA probes 8 and 13, and insulating film 2, and the transistor portion and the antenna portion are protected by the insulating film 2. A signal measured by the biomolecule detecting transistor can be transmitted via the antenna to an external receiver for signal processing. Because the antenna is embedded in the insulating film 2, it does not come into direct contact with the sample solution, so that the antenna is not subject to corrosion due to solutions or characteristics changes due to the adsorption of proteins. Thus, the biomolecule detecting transistor can be suitably used for highly reliable measurement.

Fig. 6 shows an example in which the biomolecule detecting transistor of the present embodiment and a chip having a wireless communications function are integrated. On a silicon substrate 1 measuring 1 mm square, a source 3 and a drain 4 of the biomolecule detecting transistor are formed, and a floating electrode 7 and the gate are connected via a lead-out electrode 6. A DNA probe 8 is immobilized on the floating electrode. On this silicon substrate, an antenna 21, a computation circuit 23, a memory circuit 24, a reception circuit 25, a transmission circuit 26, and a power supply circuit 27 have been integrated. If a target DNA with a complementary sequence exists in a sample, it hybridizes to the DNA probe on the floating electrode 7, forming a double strand. The formation of the double strand is detected by a field effect transistor. In the memory circuit 24 are stored ID information distinguishing one chip substrate from another, DNA probe sequence information, and coding protein information, for example. These information, as well as the information regarding the result of hybridization reaction, are transmitted to an outside reception device via the antenna and transmission circuit. Electromagnetic wave transmitted from outside the chip is received by the antenna and converted through the reception circuit and the power supply circuit into sufficient power that can be used by the chip. The power is then fed to the individual elements including the field effect transistor, transmission circuit, and memory circuit for their operation.

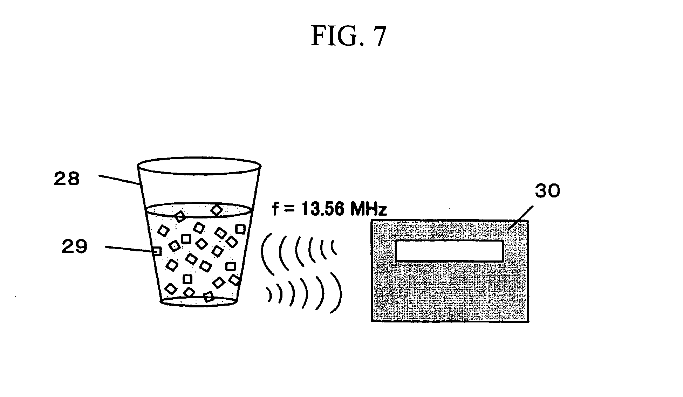

In the present embodiment, because the information identifying each chip can be acquired simultaneously with the result of hybridization, a plurality of chips can be reacted in the sample simultaneously, as shown in Fig. 7. Initially, the biomolecule detecting transistor 29 of the invention and 0.5 ml of a buffer solution are put in a reaction vessel 28, and transistor signals are measured. The measured signals are transmitted to an external receiver, and then a gene analysis is carried out in accordance with the following steps (a) to (f):
(a) A sample solution containing at least one kind of DNA is introduced into the reaction vessel, and hybridization with a single-stranded DNA probe on a conductive electrode is carried out at a predetermined temperature.
(b) A washing solution is introduced into the reaction vessel and unreacted DNA on the substrate is removed.
(c) An intercalator solution is introduced into the reaction vessel and caused to react with a double-stranded DNA.
(d) A washing solution is introduced into the reaction vessel and unreacted intercalator on the substrate is removed.
(e) A buffer solution is introduced into the reaction vessel, and output values of the insulated gate field effect transistor are measured.
(f) Output values are transmitted to the receiver via antenna.

The difference in output values of the biomolecule detecting transistor obtained by the above-described two measurements constitutes the signal from the double-stranded DNA formed by hybridization. For the measurement of signals from the biomolecule detecting transistor, electromagnetic wave of 13.56MHz is used, for example, between the transistor and an externally located transmission/reception apparatus 30. By referring to the information stored in the memory circuit, the type of DNA existing in the sample, its shape, and its sequence can be analyzed. Furthermore, because the present chip employs wireless communications technology for transmission/reception and for the supply of power, the chip can be directly put in a sample for measurement without requiring any wiring between the chip and external circuitry. Thus, a simple measurement system can be constructed. Depending on experimental conditions, the washing steps (b) and (d) may be omitted and the entire measurement sequence can be completed with the biomolecule detecting transistor immersed in the sample solution.

## Claims

1. A biomolecule detecting element comprising:
an insulated gate field effect transistor having a gate electrode embedded in an insulating film;
a probe-immobilized electrode formed on the surface of said insulating film and having a biomolecular probe immobilized thereon; and
a connection wire for electrically connecting said gate electrode and said probe-immobilized electrode,
wherein the region of said probe-immobilized electrode where said biomolecular probe is immobilized is located away from immediately above said gate electrode.

2. The biomolecule detecting element according to claim 1, wherein said probe-immobilized electrode extends from immediately above said gate electrode along the film surface of said insulating film to the region where said biomolecular probe is immobilized, wherein said connection wire is connected to said probe-immobilized electrode immediately above said gate electrode.

3. The biomolecule detecting element according to claim 1, wherein said probe-immobilized electrode is disposed away from immediately above said gate electrode, and wherein said connection wire is disposed within said insulating film along the film surface.

4. The biomolecule detecting element according to any one of claims 1 to 3, wherein said biomolecular probe comprises a nucleic acid, polynucleotide, or synthetic oligonucleotide.

5. The biomolecule detecting element according to any one of claims 1 to 4, wherein said probe-immobilized electrode is comprised of one or a combination of materials selected from gold, platinum, palladium, titanium, chromium, aluminum, polysilicon, tantalum, or molybdenum.

6. The biomolecule detecting element according to any one of claims 1 to 5, wherein a transmission/reception antenna is formed in said insulating film.

7. A biomolecule detecting element comprising:
a plurality of insulated gate field effect transistors each having a gate· electrode embedded in a common insulating film;
a plurality of probe-immobilized electrodes formed on the surface of said insulating film and having biomolecular probes immobilized thereon; and
a plurality of connection wires for electrically connecting the gate electrode of each of said plurality of insulated gate field effect transistors and said plurality of probe-immobilized electrodes,
wherein the region of said probe-immobilized electrodes where said biomolecular probes are immobilized is located away from immediately above said gate electrode.

8. The biomolecule detecting element according to claim 7, wherein a transmission/reception antenna is formed in said common insulating film.

9. The biomolecule detecting element according to claim 6 or 8, further comprising a computation circuit, a memory circuit, a reception circuit, a transmission circuit, and a power supply circuit.

10. The biomolecule detecting element according to claim 9, wherein said power supply circuit converts an electromagnetic wave received by said antenna into electric power and feeds it to individual portions.

11. A method for analyzing nucleic acids using the biomolecule detecting element according to any one of claims 1 to 10, comprising the steps of:
immobilizing a single-stranded nucleic acid probe on said probe-immobilized electrode as said biomolecular probe;
introducing a sample solution containing at least one kind of nucleic acid onto said biomolecule detecting element and carrying out hybridization with said single-stranded nucleic acid probe;
introducing a washing solution onto said biomolecule detecting element and removing unreacted nucleic acid on said biomolecule detecting element;
introducing an intercalator solution onto said biomolecule detecting element and causing it to react with the nucleic acid that has become double-stranded;
introducing a washing solution onto said biomolecule detecting element and removing unreacted intercalator on said biomolecule detecting element; and
introducing a buffer solution onto said biomolecule detecting element and measuring output values of said insulated gate field effect transistor.

12. A method for analyzing biomolecules using the biomolecule detecting element according to claim 10, comprising the steps of:
putting a plurality of biomolecule detecting elements comprising said probe-immobilized electrodes having different kinds of single-stranded nucleic acid probes immobilized thereon as biomolecular probes, and a buffer solution in a reaction vessel, and receiving a signal from each of said biomolecule detecting elements using an external receiver;
introducing a sample solution containing at least one kind of nucleic acid into said reaction vessel and carrying out hybridization with said single-stranded nucleic acid probe;
introducing an intercalator solution into said reaction vessel and causing it to react with the nucleic acid that has become double-stranded; and
receiving a signal from each of said biomolecule detecting elements using the external receiver.
